# EUROPEAN PATENT SPECIFICATION

(11) **EP 4 460 735 B1**
(45) Date of publication and mention of the grant of the patent: **03.06.2026**
(21) Application number: 23704479.7
(22) Date of filing: 04.01.2023
(51) Int. Cl.: G05D 7/06, B67C 3/20, B65B 3/00, B65B 3/12, B65B 3/36, B65B 39/12, B65B 43/52, B65B 43/56, B65B 57/14

(54) **CHARACTERIZATION AND METHOD TRANSFER OF DRUG PRODUCT FILLING RECIPES**
CHARAKTERISIERUNG UND VERFAHRENTRANSFER VON ARZNEIMITTELPRODUKTFÜLLREZEPTUREN
CARACTÉRISATION ET PROCÉDÉ DE TRANSFERT DE RECETTES DE REMPLISSAGE DE PRODUIT MÉDICAMENTEUX

(30) Priority: 04.01.2022 US 202263296361 P
(43) Date of publication of application: 13.11.2024
(73) Proprietor: AMGEN INC., Thousand Oaks, CA 91320-1799 (US)
(72) Inventor: KRUCHOWY, Evan, Thousand Oaks, CA 91320-1799 (US); MATHUR, Ian, Thousand Oaks, CA 91320-1799 (US); PADMAKUMAR, Vikashni, Thousand Oaks, CA 91320-1799 (US); LE, David, Thousand Oaks, CA 91320-1799 (US); BELLENFANT, Tyler, Thousand Oaks, CA 91320-1799 (US); WHETSTONE, Sarah, Madeleine, Thousand Oaks, CA 91320-1799 (US)
(74) Representative: J A Kemp LLP
(86) International application number: PCT/US2023/010094
(87) International publication number: WO 2023/133126

(56) References cited:
- US-A1- 2015 114 515
- US-A1- 2016 297 660
- US-A1- 2019 127 198
- US-B1- 7 661 289

## Description

### FIELD OF THE DISCLOSURE

The present application relates generally to a method and a system for characterizing and/or aligning product fill recipes. The present application may relate to characterizing and/or aligning product fill recipes for use in pharmaceutical drug product filling systems.

### BACKGROUND

Product filling systems may be used for filling a container with solid, liquid, and/or gaseous product. Product filling systems may be manual (e.g., operated by a hand lever used to pump product through a tip), semi-automatic (e.g., operated by pumps controlled by an operator), or automatic (e.g., operated by pumps controlled by a computer system). Product filling systems may also be used across a variety of disciplines and industries, including, for example, life sciences/engineering, chemical sciences/engineering, medical sciences/engineering, mechanical sciences/engineering, food sciences/engineering, beverage sciences/engineering, as well as manufacturing and assembly corresponding to the aforementioned disciplines and industries.

Product filling systems are often used in pharmaceutical development, pharmaceutical testing and trials, and pharmaceutical production. A pharmaceutical liquid filling system is one type of product filling system. Pharmaceutical liquid filling systems often increase the speed and accuracy by which liquid drug suspensions may be created and are used by many pharmaceutical firms, ranging in operation size. These pharmaceutical liquid filling systems exist in many formats, including small bench tops to large scale machines, and may accommodate many different liquid viscosities. Considerations for the selection of a pharmaceutical liquid filling system include the type of liquid, handling considerations, required throughput, and the operation's manufacturing and maintenance budget.

In pharmaceutical use, there is often more than one product filling system in use, each product filling system corresponding to one or more filling recipes. Multiple product filling systems may be used, for example, because one smaller product filling system is used during drug development and another larger product filling system is used during drug manufacturing. In another example, multiple product filling systems may be used during drug manufacturing to, for example, increase overall manufacturing throughput. However, it may be undesirable or even impossible to use multiple product filling systems if the operation of the multiple product filling systems produces differing filling outcomes. Therefore transferability between filling recipes may be critical to pharmaceutical use of multiple product filling systems. Transferability may be achieved between multiple product filling systems, for example, if the multiple product filling systems produce the same outcome when filling containers with product. The outcomes may be considered the same if, for example, the containers all have an equivalent volume or mass of product, within a threshold. The outcomes may be considered the same if, for example, the containers all have product in the same state (e.g., undamaged, non-bubbling, etc.).

Parameters that are included in a filling recipe may be associated with: start pump dosing, dosing start ramp, dosing stop ramp, end pump dosing, drip retraction, and main drive speed. Other factors that affect the filling recipe include the hardware components used in the product filling system, including, for example: a pump, tubing, a container, a conveyor system, a tip, *etc*.

Conventionally, to attempt to improve transferability, pharmaceutical operators may purchase multiple instances of the same product filling system from a common vendor. Even still, differences in filling outcomes may exist between the instances of the same product filling system from the common vendor. In some examples, non-transferability between filling recipes may block operation of drug filling all together. In other examples, fewer product filling systems than desired may have to be used when there is non-transferability between certain instances of product filling systems. In other examples, pharmaceutical operators may attempt to standardize the instances of the product filling systems; however, an efficient and effective method of achieving transferability does not conventionally exist. Conventional methods of standardizing product filling systems to achieve transferable filling recipes have a long lead time, high cost, may be operator expertise-dependent (therefore may be inconsistently applied), and still may not achieve desired transferability. US 2016/0297660 A1 discloses a machine, a system, and a method for filling a container with a pourable product. US 2019/127198 A1 discloses a container-filling method.

### BRIEF SUMMARY

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes. One aspect of the present disclosure provides a method for characterizing and/or aligning product fill recipes, including: (a) generating, by one or more flow sensors, first flow rate data over a plurality of first fill instances corresponding to a first product fill recipe; (b) generating, by one or more processors, a plurality of first fill instance curves, wherein each first fill instance curve is a segment of the first flow rate data that corresponds to a different first fill instance of the plurality of first fill instances; (c) generating, by the one or more processors, a first flow profile based on the plurality of first fill instance curves; and (d) causing, by the one or more processors, a display to present the first flow profile.

In some embodiments, the previous aspect further include: (a) generating, by the one or more flow sensors or one or more other flow sensors, second flow rate data over a plurality of second fill instances corresponding to a second product fill recipe; (b) generating, by the one or more processors, a plurality of second fill instance curves, wherein each second fill instance curve is a segment of the second flow rate data that corresponds to a different second fill instance of the plurality of second fill instances; (c) generating, by the one or more processors, a second flow profile based on the plurality of second fill instance curves; and (d) causing, by the one or more processors, the display to present the second flow profile.

In some embodiments, the previous aspects further include determining, based on the first flow profile and the second flow profile, adjustments to one or more parameters of the second product fill recipe.

The previous aspects further include changing the one or more parameters of the second product fill recipe in accordance with the adjustments.

Another aspect of the present disclosure provides a system including: (a) one or more flow sensors; (b) one or more processors; and (c) one or more non-transitory, computer-readable media storing instructions that, when executed by the one or more processors, cause the one or more processors to perform the method of any one of the previous aspects.

### BRIEF DESCRIPTION OF THE DRAWINGS

The skilled artisan will understand that the figures described herein are included for purposes of illustration and are not limiting on the present disclosure. The drawings are not necessarily to scale, emphasis instead being placed upon illustrating the principles of the present disclosure. It is to be understood that, in some instances, various aspects of the described implementations may be shown exaggerated or enlarged to facilitate an understanding of the described implementations. In the drawings, like primary characters throughout the various drawings generally refer to functionally similar and/or structurally similar components.
FIG. 1 is a simplified block diagram of an example system for characterizing and/or aligning product fill recipes.
FIG. 2 depicts an example product filling system.
FIG. 3 depicts example flow rate data over a single fill instance.
FIG. 4 is a flow diagram 400 depicting example data used in generating a flow profile from flow rate data over a plurality of fill instances
FIG. 5 is a flow diagram depicting an example method of causing the simultaneous display of two fill profiles corresponding to two product filling systems.
FIG. 6A is a flow diagram depicting an example method for causing a display of a first flow profile.
FIG. 6B is a flow diagram depicting an example method for causing a display of a second flow profile.

### DETAILED DESCRIPTION

The present disclosure aims to reduce problems with conventional approaches (e.g., as described in the Background section) by providing improved method(s) for characterizing and/or aligning product fill recipes. The method(s) may include: (i) generating first flow rate data over a plurality of first fill instances corresponding to a first product fill recipe, (ii) generating a plurality of first fill instance curves, (iii) generating a first flow profile based on the plurality of first fill instance curves, and (iv) causing a display to present the first flow profile. Via generating and displaying the first flow profile associated with the first product fill recipe, the method aims to characterize the first product fill recipe such that is allows for transferability to be assessed and achieved, avoiding the numerous disadvantages associated with non-transferability between multiple fill recipes.

Furthermore, in some embodiments, the disclosed method(s) may be partially or entirely automated, thereby not only improving efficiency with respect to human labor, but also removing inconsistencies due to reliance on operator technique.

The various concepts introduced above and discussed in greater detail below may be implemented in any of numerous ways, and the described concepts are not limited to any particular manner of implementation. Examples of implementations are provided below for illustrative purposes.

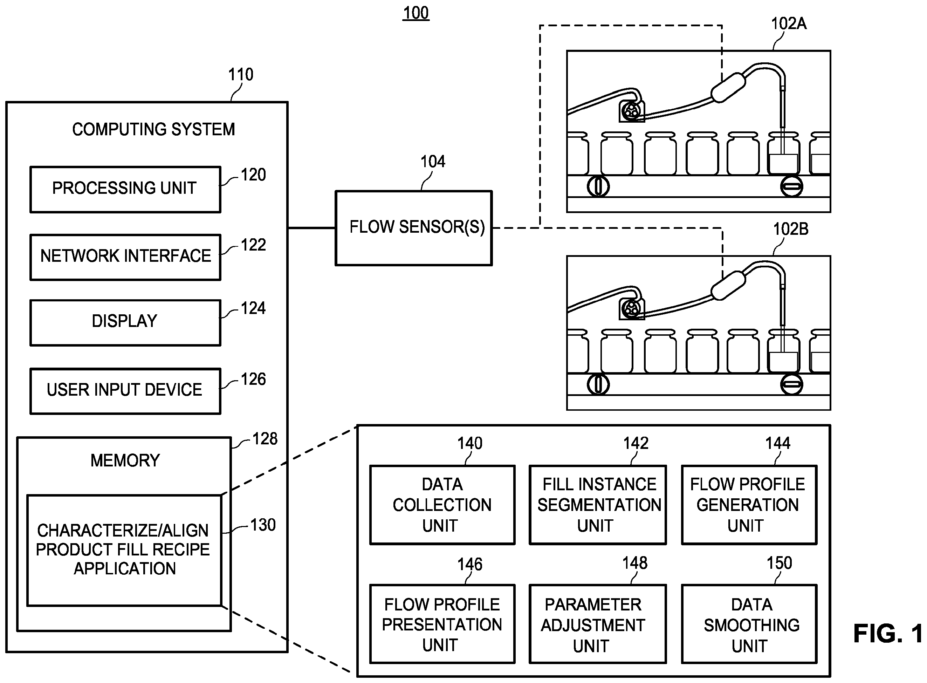

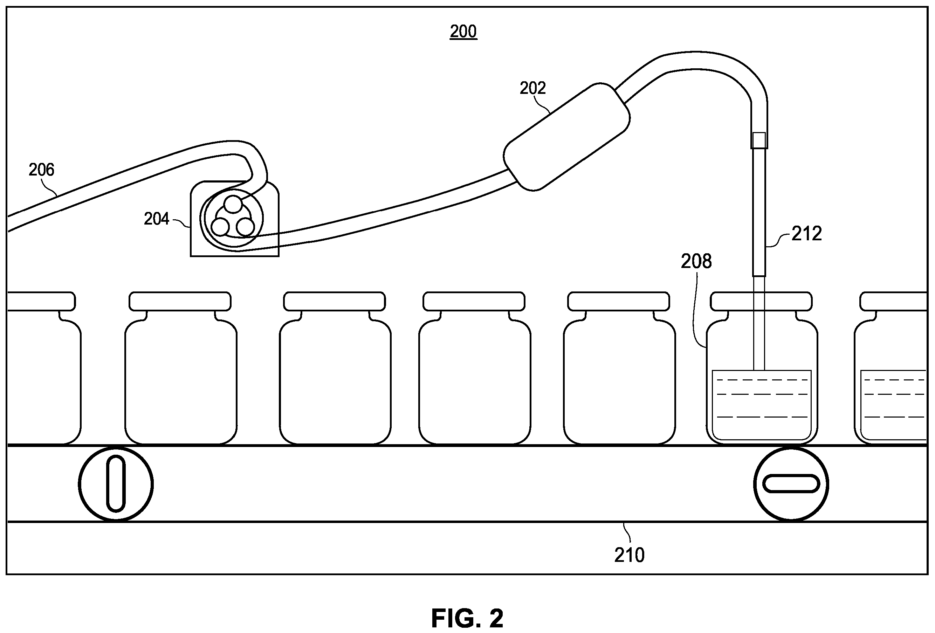

FIG. 1 is a simplified block diagram of an example system 100 for characterizing and/or aligning product fill recipes corresponding to product filling systems. In some embodiments, the product filling systems 102A and 102B may be standalone equipment, though in other examples the product filling systems 102A and 102B may be incorporated into other equipment. Product filling systems 102A and 102B may be physical devices configured for use in product filling. More specific examples of processes in which the product filling systems 102A and 102B may be used include: drug filling, chemical filling, biological matter filling, *etc.* In other embodiments, the system 100 is equipment that is used in a process unrelated to pharmaceutical development or production (e.g., a food or beverage manufacturing plant, an oil processing plant, *etc.).* A more detailed example and description of a product filling system is provided in FIG. 2 and its corresponding description. In some embodiments, the product filling systems 102A and 102B are the same model and/or produced or provided by the same vendor. In other embodiments, the product filling systems 102A and 102B are different models and/or produced or provided by different vendors.

The system 100 also includes one or more flow sensors 104, which are configured to measure flow rate associated with the product filling systems 102A and 102B. For example, the flow sensor(s) 104 may measure milliliters per second of product flow in the product filling systems 102A and 102B during operation. The flow sensor(s) 104 may be ultrasonic flow sensors which measure flow rate by transmitting and receiving a burst of ultrasound. The flow sensor(s) 104 may be another type of sensor that directly measures flow rate. Alternatively, the flow sensor(s) 104 may measure flow rate indirectly (e.g., measuring volume of product in a filled container). The flow sensor(s) 104 may include one or more devices integrated on or within the product filling systems 102A and 102B, and/or one or more devices affixed to or otherwise placed in proximity with the product filling systems 102A and 102B. Depending on the embodiment, none, some, or all of the flow sensor(s) 104 may be viewed as a part of the product filling systems 102A and 102B. In particular, in embodiments where the performance of any or all of the flow sensor(s) 104 is included in the equipment performance analysis (as described further below), references herein to "the product filling systems 102A and 102B" includes those flow sensor(s) 104. For example, an analysis of the performance of characterizing and/or aligning product fill may include also analyzing the performance of the flow sensor(s) 104.

The system 100 also includes a computing system 110 coupled to the flow sensor(s) 104. As discussed in further detail below, the computing system 110 may include a single computing device, or multiple computing devices (e.g., one or more servers and one or more client devices) that are either co-located or remote from each other. The computing system 110 is generally configured to: (a) generate, by the one or more flow sensors 104, flow rate data over a plurality of fill instances corresponding to a product fill recipe; (b) generate a plurality of fill instance curves, wherein each fill instance curve is a segment of the flow rate data that corresponds to a different fill instance of the plurality of fill instances; (c) generating a flow profile based on the plurality of fill instance curves; and (d) cause a display to present the flow profile. In the example embodiment shown in FIG. 1, the computing system 110 includes a processing unit 120, a network interface 122, a display 124, a user input device 126, and a memory 128.

The processing unit 120 includes one or more processors, each of which may be a programmable microprocessor that executes software instructions stored in the memory 128 to execute some or all of the functions of the computing system 110 as described herein. Alternatively, one or more of the processors in the processing unit 120 may be other types of processors (e.g., application-specific integrated circuits (ASICs), field-programmable gate arrays (FPGAs), *etc.).*

The network interface 122 may include any suitable hardware (e.g., front-end transmitter and receiver hardware), firmware, and/or software configured to use one or more communication protocols to communicate with external devices and/or systems (e.g., the sensor device(s) 104, or a server, not shown in FIG.1, that provides an interface between the computing system 110 and the sensor device(s) 104, *etc.).* For example, the network interface 122 may be or include an Ethernet interface. While not shown in FIG. 1, the computing system 110 may communicate with the flow sensor(s) 104, and/or with any device(s) that provide an interface between the computing system 110 and the flow sensor(s) 104, via a single communication network, or via multiple communication networks of one or more types (e.g., one or more wired and/or wireless local area networks (LANs), and/or one or more wired and/or wireless wide area networks (WANs) such as the Internet or an intranet, *etc.).*

The display 124 may use any suitable display technology (e.g., LED, OLED, LCD, *etc.)* to present information to a user, and the user input device 126 may be a keyboard or other suitable input device. In some embodiments, the display 124 and the user input device 126 are integrated within a single device (e.g., a touchscreen display). Generally, the display 124 and the user input device 126 may combine to enable a user to interact with graphical user interfaces (GUls) or other (e.g., text) user interfaces provided by the computing system 110, e.g., for purposes such as displaying one or more flow profiles, displaying parameters, recommending changes to one or more parameters, notifying users of equipment faults or other deficiencies, *etc.*

The memory 128 may include one or more physical memory devices or units containing volatile and/or non-volatile memory, and may include memories located in different computing devices of the computing system 110. Any suitable memory type or types may be used, such as read-only memory (ROM), solid-state drives (SSDs), hard disk drives (HDDs), and so on. The memory 128 stores the instructions of one or more software applications, including a characterize/align product fill recipe application 130. The characterize/align product fill recipe application 130 includes a data collection unit 140, a fill instance segmentation unit 142, a flow profile generation unit 144, a flow profile presentation unit 146, a parameter adjustment unit 148, and a data smoothing unit 150. The units 140 through 150 may be distinct software components or modules of the characterize/align product fill recipe application 130, or may simply represent functionality of the characterize/align product fill recipe application 130 that is not necessarily divided among different components/modules. For example, in some embodiments, the fill instance segmentation unit 142 and the data smoothing unit 150 are included in a single software module. Moreover, in some embodiments, the different units 140 through 150 may be distributed among multiple copies of the characterize/align product fill recipe application 130 (*e.g.,* executing at different devices in the computing system 110), or among different types of applications stored and executed at one or more devices of the computing system 110. The operation of each of the units 140 through 150 is described in further detail below, with reference to the operation of the system 100.

FIG. 2 depicts an example product filling system 200. The system 200 illustrated in FIG. 2 may be standalone equipment, such as a VarioSys ^{®} filling machine, though in other examples the system 200 may be incorporated into other equipment. The system 200 may be used for filling containers with product which may include liquids, solids, gases, or plasmas (*e.g.,* drug products).

In this example, the system 200 includes a flow sensor 202 for measuring flow rate of the product, a pump 204 to pump the product, tubing 206 for the product to flow through, containers 208 to receive the product, a conveyor system 210 to move the containers 208, and a tip 212 at which the product exits the tubing 206.

The flow sensor may measure an amount of the product flowing through the tubing 206 in a given time interval. For example, the flow sensor 202 may measure milliliters of product flowing through the tubing 206 per second. The flow sensor 202 may be an ultrasonic flow sensor which measures flow rate by transmitting and receiving a burst of ultrasound. Alternatively, the flow sensor 202 may be a mechanical flow sensor. The flow sensor 202 may be another type of sensor that directly measures flow rate. Alternatively, the flow sensor 202 may measure flow rate indirectly (*e.g.,* measuring volume of product in a filled container). The flow sensor 202 may include one or more devices integrated on or within the system 200 and/or one or more devices affixed to or otherwise placed in proximity with the system 200. For example, the flow sensor 202 may be integrated into one or more of: the pump 204, the tubing 206, the containers 208, or the tip 212.

The pump 204, may pump the product through the tubing 206, out the tip 212, and into the containers 208. The pump 204 may be a positive-displacement pump, such as a peristaltic pump. Alternatively, the pump 204 may be at least one of: an impulse pump, a velocity pump, a gravity pump, a steam pump, or a valveless pump. The pump 204 may have a main drive speed that is controllable via a controller and/or an operator. The controller and/or operator may instruct the pump 204 when to begin start pump dosing (*e.g.,* once an empty container is positioned under the tip 212) and when to begin end pump dosing (*e.g.,* to allow for a desired amount of product to fill the containers 208). The pump 204 may be controlled by the one or more processors (such as the one or more processors 120 of FIG. 1). The main drive speed may be fixed or it may be variable during operation of the pump 204. The main drive speed of the pump 204 may be a parameter of a product filling recipe that is adjustable. In adjusting the main drive speed of the pump 204, it is important to not increase the main drive speed beyond a threshold, as beyond the threshold, the product (particularly for fluid products) may splash, become agitated, bubble, or have losses on the sides of the containers 208.

The tubing 206 may be of variable or fixed diameter and flexible or rigid in construction. Examples of possible tubing materials may include: polyethylene, nylon, urethane, copper, stainless steel, plastic, polyvinyl chloride, silicone rubber, thermoplastic, fluoroelastomer, *etc.* The diameter, construction, materials, or other tubing parameters of the tube 206 may be dependent on the pump 204, including, for example, the main drive speed of the pump. Therefore, the tube 206 may be selected as part of a product filling recipe to allow for certain adjustments to parameters of the product filling recipe, such as the main drive speed.

The containers 208 may be of a variety of different types, for example the containers 208 may be at least one of: vials, syringes, cartridges, tubes, beakers, cups, or any other suitable holding structure for the product. In some embodiments, more than one container of the containers 208 may be filled at once (*i.e.,* a product filling system may be configured to fill a plurality of containers of the containers 208 simultaneously). Different types of containers 208 may correspond to different main drive speed thresholds at which increasing the main drive speed above the threshold, may cause the product to (particularly for fluid products) splash, become agitated, bubble, or have losses on the side of the containers 208. Therefore, the containers 208 may be selected as part of a product filling recipe to allow for certain adjustments to parameters of the product filling recipe, such as the main drive speed.

The conveyor system 210, for moving the containers 208 into position with respect to the tip 212, may be of fixed or variable speed. The conveyor system 210 may be controlled by the one or more processors (such as the one or more processors 120 of FIG. 1). The type of conveyor system 210 or parameters (*e.g.,* speed) of the conveyor system 210 may be selected based on the parameters of the product filling recipe. For example, parameters of the product filling recipe, such as dosing start ramp or dosing stop ramp may affect the type of conveyor system 210 or the parameters of the conveyor system 210.

The tip 212 may be for example, a sprayer, a pipette tip, a dropper, a nozzle, or some other device suitable for facilitating ejection of the product from the tubing 206 and into the containers 208. The tip 212 may be capable of being used with drop retraction of the product, preventing a final drop of product from being ejected into the containers 208. Accordingly, the tip 212 may be selected as part of a product filling recipe to allow for certain adjustments to parameters of the product filling recipe, such as the drop retraction.

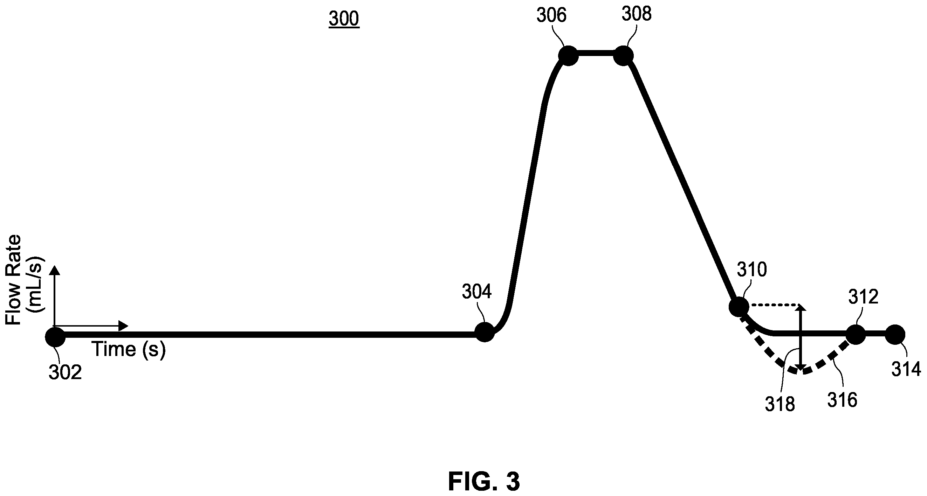

FIG. 3 depicts example flow rate data over a single fill instance 300. Generally, the example experimental results included in FIG. 3 may be used in generating a flow profile and accordingly, characterizing and/or aligning product fill recipes.

The example experimental data included in FIG. 3 may correspond to a single fill instance performed by a product filling system, such as the product filling system 200 of FIG. 2.

In FIG. 3, the flow rate (mL/s) of the single fill instance is plotted as a function of time (measured in seconds). Marked on the flow rate curve are the following: a start time 302, a start pump dosing point 304, a dosing start ramp point 306, a dosing stop ramp point 308, an end pump dosing point 310, an end drip retraction point 312, and an end time 314. Also marked in FIG. 3 are a drip retraction curve 316 and a drip retraction distance 318.

The start time 302 may be the time in which flow rate data begins being collected. The start time 302 may correspond to powering on or starting data collection by a flow rate sensor (which may be the same as or similar to the flow sensor 202 of FIG. 2 and/or the flow sensor(s) 104 of FIG. 1).

The start pump dosing point 304 may correspond to a pump beginning operation, which may be the same as or similar to the pump 204 of FIG. 2. For the embodiment of a peristaltic pump, the start pump dosing point 304 may correspond to the peristaltic pump just beginning to rotate. The time in which the start pump dosing point 304 occurs may be a parameter of a product filling recipe that is adjustable.

The region between the start pump dosing point 304 and the dosing start ramp point 306, may correspond to the pump increasing pumping rate from zero (at the start pump dosing point 304) up to a main drive speed (at the dosing start ramp point 306). For the embodiment of the peristaltic pump, this region may correspond to the peristaltic pump increasing from zero rotations per minute up to a fixed number of rotations per minute (*i.e.,* the main drive speed). The behavior (*e.g.,* rate of increasing pumping rate, length of time in which pump increases pumping rate, pattern of increasing pumping rate, *etc.*) of the pump in increasing from zero up to the main drive speed may include parameters of a product filling recipe that are adjustable.

The region between the dosing start ramp point 306 and the dosing stop ramp point 308, may correspond to the pump operating at the main drive speed with little or no change in pumping rate. For the embodiment of the peristaltic pump, this region may correspond to the peristaltic pump operating at the fixed number of rotations per minute (*i.e.,* the main drive speed). The main drive speed and/or a length of time in which the pump operates at the main drive speed may be parameters of a product filling recipe that are adjustable.

The region between the dosing stop ramp point 308 and the end pump dosing point 310, may correspond to the pump decreasing pumping rate from the main drive speed (at the dosing stop ramp point 308) down to zero or near zero (at the end pump dosing point 310). For the embodiment of the peristaltic pump, this region may correspond to the peristaltic pump decreasing from a fixed number of rotations per minute (*i.e.,* the main drive speed) down to zero rotations per minute. The behavior (*e.g.,* rate of decreasing pumping rate, length of time in which pump decreases pumping rate, pattern of decreasing pumping rate, *etc*.) of the pump in decreasing from the main drive speed to zero may include parameters of a product filling recipe that are adjustable.

The end pump dosing point 310 may correspond to a pump ending operation. For the embodiment of the peristaltic pump, the end pump dosing point 310 may correspond to the peristaltic pump ending rotation. The time in which the end pump dosing point 310 occurs may be a parameter of a product filling recipe that is adjustable.

The end time 312 may be the time in which flow rate data stops being recorded. The end time 312 may correspond to powering off or stopping data collection by the flow rate sensor.

As depicted in FIG. 3, the end pump dosing point 310 does not correspond to exactly zero flow rate. This may be due to a residual amount of product still leaving at least one of the pump, a tube (which may be the same as or similar to the tube 206 of FIG. 2), or a tip (which may be the same as or similar to the tip 212 of FIG. 2) after the pump has ended operation. Accordingly, in some embodiments, drip retraction is used to counteract the residual amount of product leaving the at least one of the pump, the tube, or the tip. The drip retraction curve 316 is depicted in FIG. 3 as having a negative flow rate, corresponding to the residual amount of product being removed prior to entering a container (which may be the same as or similar to the containers 208 of FIG. 2). Also depicted in FIG. 3 is a drip retraction distance 318 corresponding to the drip retraction curve. Whether or not to use drip retraction may be a parameter of a product filling recipe that is adjustable. Furthermore, the behavior (*e.g.,* the drip retraction curve 316 shape, the drip retraction distance 318, *etc.)* of performing the drip retraction may include parameters of a product filling recipe that are adjustable.

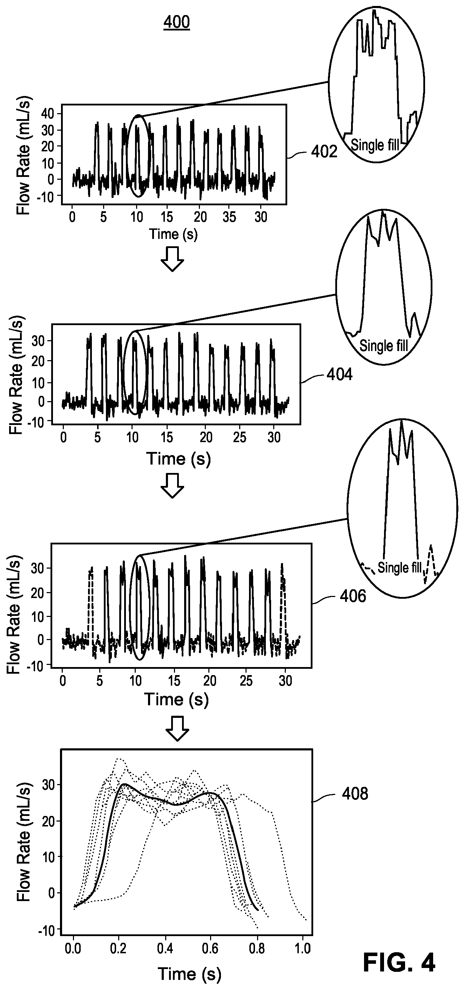

FIG. 4 is a flow diagram 400 depicting example data used in generating a flow profile from flow rate data over a plurality of fill instances. The example experimental data included in FIG. 4 may correspond to thirteen fill instances performed by a product filling system, such as the product filling system 200 of FIG. 2.

In FIG. 4 flow rate (mL/s) of the fill instances are plotted as a function of time (measured in seconds). Four stages of data are shown in FIG. 4 for generating the flow profile from the flow rate data, this includes: the flow rate data 402, smoothed flow rate data 404, segmented smoothed flow rate data 406, and the flow profile 408. Generating at least one of the four stages of data may include using a machine learning algorithm.

The flow rate data 402 may correspond to the product filling system 200 of FIG. 2, or some other product filling system and may have been generated and/or collected using a flow rate sensor (which may be the same as or similar to the flow sensor 202 of FIG. 2 and/or the flow sensor(s) 104 of FIG. 1) and a data collection unit (which may be the same as or similar to the data collection unit 140 of FIG. 1). As depicted in FIG. 4, there are thirteen fill instances which the flow rate data 402 includes. Between each of the fill instances there may be noise. All thirteen of the fill instances may correspond to a single product filling system (which may be the same as or similar to the product filling system 200 of FIG. 2). Although each of the thirteen fill instances may correspond to a single product filling system, there may still be some variability in parameters of the single product filling system during the thirteen fill instances, such as variability in one or more of: a start pump dosing point, a dosing start ramp point, a dosing stop ramp point, an end pump dosing point, an end drip retraction point, a main drive speed, *etc.* A zoomed window depicts a single fill instance of the thirteen fill instances included in the flow rate data 402. The zoomed window of 402 depicts clipping, chopping, and/or other possibly undesirable characteristics of the flow rate data 402.

The smoothed flow rate data 404 may be smoothed data corresponding to the flow rate data 402 and may be generated using a data smoothing unit (which may be the same as or similar to the data smoothing unit 150 of FIG. 1). The smoothed flow rate data 404 may smooth the clipping, chopping, and/or other possibly undesirable characteristics of the flow rate data 402. Smoothing the flow rate data 402 to generate the smoothed flow rate data 404 may be done through, for example, creating an approximating function to capture important patterns in the flow rate data 402, while leaving out noise or other fine-scale structures/rapid phenomena (*i.e.,* data points of the flow rate data 402 are modified so individual points higher than adjacent points (possibly because of noise) are reduced, and data points that are lower than adjacent points (possibly because of noise) are increased) leading to a smoother signal. The smoothed flow rate data 404 presents advantages over the flow rate data 402, such as it may be possible to extract more information from the smoothed flow rate data 404 (provided a reasonable smoothing), and it may be possible to perform analyses on the smoothed flow rate data 404 that are flexible and robust. The zoomed window of 404 depicts smoother flow rate data than depicted in the zoomed window of 402.

The segmented smoothed flow rate data 406 may be segmented data corresponding to the smoothed flow rate data 404 and may be generated using a fill instance segmentation unit (which may be the same as or similar to the fill instance segmentation unit 142 of FIG. 1). The segmented smoothed flow rate data 406 may segment each of the thirteen fill instances included in the flow rate data 402 and smoothed flow rate data 404. Each of the thirteen fill instances may be segmented to include only data corresponding to between the start pump dosing point and the end pump dosing point for each fill instance. The segmented fill instances are shown as solid lines, whereas the data points excluded from the segmented smoothed flow rate data 406 are shown as dashed lines. Identifying each fill instance for segmenting the smoothed flow rate data 404 may be done based on factors or features of the smoothed flow rate data 404, including, for example, one or more of: prominence of fill instance peaks, minimum spacing between fill instances, minimum height of the fill instance peaks, minimum threshold of the fill instance peaks, or minimum width of the fill instance peak. Accordingly, data points between fill instances may be excluded from the segmented smoothed flow rate data 406. By segmenting the smoothed flow rate data 404 into thirteen fill instances, each fill instance may be individually analyzed. In some embodiments, a beginning and an ending fill instance may be excluded from the segmented flow rate data 406, as shown in FIG. 4. Excluding the beginning and the ending fill instance may be done to improve accuracy and the representation of the data with respect to the corresponding product fill system.

The flow profile 408 may be generated based on the segmented smoothed flow rate data 406 using a flow profile generation unit (which may be the same as or similar to the flow profile generation unit 144 of FIG. 1). More specifically, the flow profile 408 depicted in FIG. 4 may be based on eleven (excluding the beginning and the ending fill instance of the thirteen fill instances of the fill instances of the segmented smoothed flow rate data 406) fill instances of the smoothed flow rate data 406. To generate the flow profile from the segmented smoothed flow rate data 406, dynamic time warping may be performed on each of the fill instances of the segmented smoothed flow rate data 406. By performing dynamic time warping, the impacts of variations in the segmentation of the fill instances on an average of the fill instances may be lessened. While many different methods for averaging each of the fill instances of the segmented smoothed flow rate data 406 after dynamic time warping has been applied may be used, in one embodiment, k-means averaging may be used. In fact, in the example data included in FIG. 4, dynamic time warping and k-means averaging are applied to each of the eleven fill instances of the segmented smoothed flow rate data 406 (shown as dashed lines) to produce the flow profile 408 (shown as a solid line) as depicted.

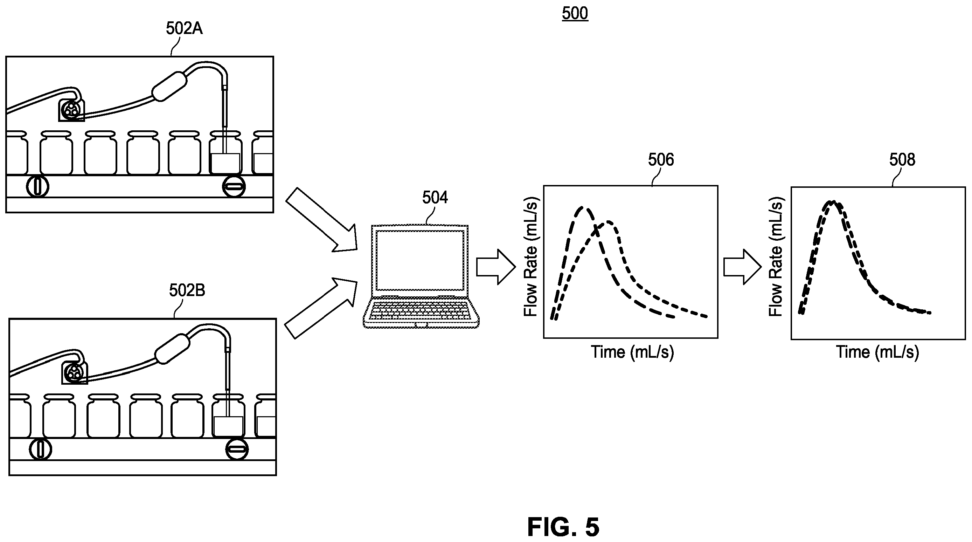

FIG. 5 is a flow diagram 500 depicting an example method for changing the parameters of a filling recipe of at least one of two product filling systems to achieve transferability between the two product filling systems. The example method may include the following steps: (1) collect flow rate data from two product filling systems (steps 502A and 502B), (2) generate two flow profiles corresponding to the flow rate data from each of the two product filling systems (step 504), (3) compare the two flow profiles to determine adjustments to parameters of a filling recipe of at least one of the two product filling systems for achieving transferability (step 506), and (4) change the parameters of the filling recipe of the at least one of the two product filling systems (step 508).

Collecting flow rate data from the two product filling systems (steps 502A and 502B) uses one or more flow sensors, such as the flow sensor(s) 104 of FIG. 1 and/or the flow sensor 202 of FIG. 2, as well as possibly the data collection unit 140 of FIG. 1. The two product filling systems may be the same model and/or produced or provided by the same vendor. In other embodiments, the two product filling systems may be different models and/or produced or provided by different vendors. At least one of the two product filling systems may be, for example, the product filling systems 102A and 102B of FIG. 1 and/or the product filling system 200 of FIG. 2.

Generating the two flow profiles corresponding to the flow rate data from each of the two product filling systems (step 504) may use a computing device, such as the computing device 110 of FIG. 1, as well as possibly one or more of: the fill instance segmentation unit 142, the flow profile generation unit 144, or the data smoothing unit 150, of FIG. 1. The computing device may, for example, perform steps similar to those corresponding to the example data used in generating a flow profile from flow rate data over a plurality of fill instances of FIG. 4. For example, the computing device may: (i) generate the flow rate data, (ii) generate smoothed flow rate data, (iii) generate segmented smoothed flow rate data, and (iv) generate the flow profile. The two flow profiles, corresponding to the flow rate data from each of the two product filling systems, may be generated in parallel or in series.

Comparing the two flow profiles to determine adjustments to parameters of a filling recipe of at least one of the two product filling systems for achieving transferability (step 506) may use a computing device which may be the same computing device used in step 504, or it may be a different computing device. The comparison (step 506) may use the computing device 110 of FIG. 1, as well as possibly the parameter adjustment unit 148 of FIG. 1. Comparing the two flow profiles to determine adjustments to parameters of a filling recipe of at least one of the two product filling systems for achieving transferability (step 506) may include causing, by the computing device, the display of the two flow profiles, possibly on a shared set of axes (which may use the flow profile presentation unit 146 of FIG. 1). The adjustments to the parameters may be associated with one or more of: start pump dosing, dosing start ramp, dosing stop ramp, end pump dosing, drip retraction, or main drive speed. Determining the adjustments may be based on a Fréchet distance between the first flow profile and the second flow profile.

Changing the parameters of the filling recipe of the at least one of the two product filling systems 508 may use a computing device which may be the same computing device used in step 504 and/or step 506, or it may be a different computing device. The changing of step 508 may use the computing device 110 of FIG. 1, as well as possibly the parameter adjustment unit 148 of FIG. 1. The parameters may be changed, for example, via an operator providing instruction to a computing system via a user input device, such as the user input device 126 of FIG. 1. Changing the parameters may include a user directly replacing and/or modifying components of one or more of the product filling systems. For example, an operator may increase the main drive speed of one of the product filling systems, and, in response to increasing the main drive speed, replace the containers of all the product filling systems to new containers suited for an increased main drive speed.

Changing the parameters of the filling recipe of the at least one of the two product filling systems 508 may further include confirming transferability has been achieved after the parameters have been changed. Confirming transferability has been achieved may include an iterative method of performing steps 502A, 502B, 504, and 506 (in part) again after the parameters have been changed; thereby (1) collecting new flow rate data from two product filling systems (steps 502A and 502B), (2) generating two new flow profiles corresponding to the flow rate data from each of the two product filling systems (step 504), and (3) comparing the two new flow profiles to confirm transferability has been achieved (step 506, in part). In the event transferability has been achieved, the method may cease as the desired condition has been met. In the event transferability has not been achieved, the method may continue with: determining new adjustments to parameters of a filling recipe of at least one of the two product filling systems for achieving transferability (step 506, in part) and changing the parameters of the filling recipe of the at least one of the two product filling systems (step 508) again. This iterative method may further continue iteratively until the condition of transferability has been met.

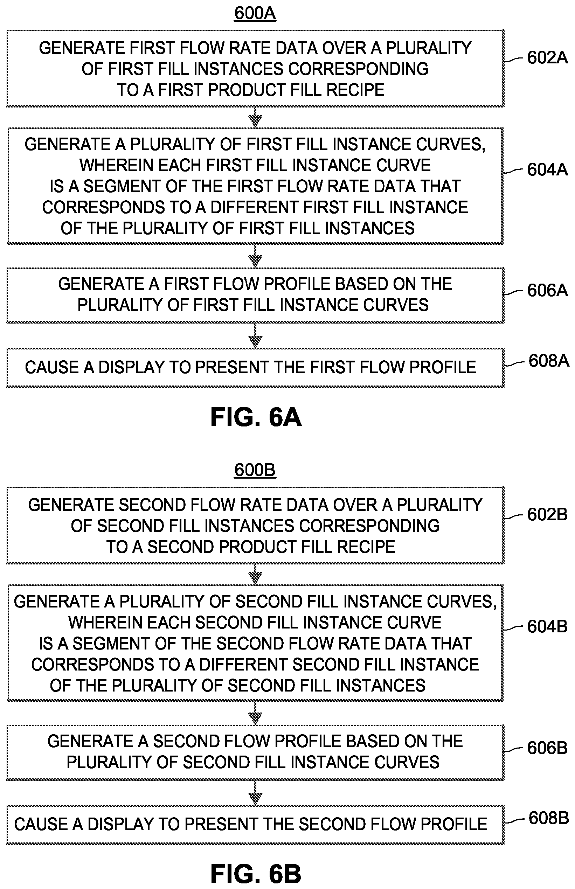

FIG. 6A and 6B are flow diagrams, each depicting an example method 600A and 600B for causing a display of a first flow profile and a second flow profile, respectively.

In the depicted method 600A/600B, first/second flow rate data are generated over a plurality of first/second fill instances corresponding to a first/second product fill recipe (block 602A/602B). For example, there may be 10 first fill instances for which first flow rate data are generated and 100 second fill instances for which second flow rate data are generated. This example may correspond to the first flow rate data corresponding to a first product filling system used in drug development and the second flow rate data corresponding to a second product filling system used in drug manufacturing, wherein the second product filling system has a larger capacity and/or throughput than the first product filling system.

Next, in the depicted method 600A/600B, a plurality of first/second fill instance curves are generated, wherein each first/second fill instance curve is a segment of the first/second flow rate data that corresponds to a different first/second fill instance of the plurality of first/second fill instances (block 604A/604B). The plurality of first/second fill instance curves may correspond to all of or some of the first/second fill instances. For example, the plurality of first/second fill instance curves may correspond to each of the first/second fill instances, except for the beginning and the ending fill instance. Segmenting the first/second flow rate data into first/second fill instances may be based on one or more of: prominence, minimum spacing, minimum height, minimum threshold, or minimum width.

Next, in the depicted method 600A/600B, a first/second flow profile is generated based on the plurality of first/second fill instance curves (block 604A/604B). For example, the first/second flow profile may be generated using dynamic time warping and/or k-means.

Finally, in the depicted method 600A/600B, a display is caused to present the first/second flow profile. In some embodiments, the first and second flow profile may be displayed separately, possibly on separate displays. In other embodiments, the first and second flow profile may be simultaneously displayed on a shared set of axes on the display. Displaying the first and second flow profile simultaneously on a shared set of axes may allow for comparison of the flow profiles by an operator.

According to non-claimed embodiments the method 600A/600B may be performed entirely by a humar operator. According to the invention, the method 600A/600B is performed entirely by automation, e.g., by one or more processors (*e.g.,* a CPU and/or GPU) that execute instructions stored on one or more non-transitory, computer-readable storage media (*e.g.,* a volatile memory or a non-volatile memory, a read-only memory, a random-access memory, a flash memory, an electronic erasable program read-only memory, and/or one or more other types of memory). The method 600A/600B may be performed entirely, or in part, by machine learning algorithms. In still other embodiments, the method 600A/600B is performed in part by a human operator, and in part by one or more processors executing instructions. For example, a human may enter an instruction (e.g., press a virtual button on a graphical user interface generated by one or more processors) to begin collecting first/second flow rate data, and in response, one or more processors may trigger one or more components of a first/second product fill system, including flow sensor(s) to begin operation. The method 600A/600B may use any of the components of FIG. 1 and/or FIG. 2.

Some of the figures described herein illustrate example block diagrams having one or more functional components. It will be understood that such block diagrams are for illustrative purposes and the devices described and shown may have additional, fewer, or alternate components than those illustrated. Additionally, in various embodiments, the components (as well as the functionality provided by the respective components) may be associated with or otherwise integrated as part of any suitable components.

Some embodiments of the disclosure relate to a non-transitory computer-readable storage medium having instructions/computer-readable storage medium thereon for performing various computer-implemented operations. The term "instructions/computer-readable storage medium" is used herein to include any medium that is capable of storing or encoding a sequence of instructions or computer codes for performing the operations, methodologies, and techniques described herein. The media and computer code may be those specially designed and constructed for the purposes of the embodiments of the disclosure, or they may be of the kind well known and available to those having skill in the computer software arts. Examples of computer-readable storage media include, but are not limited to: magnetic media such as hard disks, floppy disks, and magnetic tape; optical media such as CD-ROMs and holographic devices; magneto-optical media such as optical disks; and hardware devices that are specially configured to store and execute program code, such as ASICs, programmable logic devices ("PLDs"), and ROM and RAM devices.

Examples of computer code include machine code, such as produced by a compiler, and files containing higher-level code that are executed by a computer using an interpreter or a compiler. For example, an embodiment of the disclosure may be implemented using Java, C++, or other object-oriented programming language and development tools. Additional examples of computer code include encrypted code and compressed code. Moreover, an embodiment of the disclosure may be downloaded as a computer program product, which may be transferred from a remote computer (*e.g.,* a server computer) to a requesting computer (*e.g.,* a client computer or a different server computer) via a transmission channel. Another embodiment of the disclosure may be implemented in hardwired circuitry in place of, or in combination with, machine-executable software instructions.

As used herein, the singular terms "a," "an," and "the" may include plural referents, unless the context clearly dictates otherwise.

As used herein, the terms "approximately," "substantially," "substantial," "roughly" and "about" are used to describe and account for small variations. When used in conjunction with an event or circumstance, the terms can refer to instances in which the event or circumstance occurs precisely as well as instances in which the event or circumstance occurs to a close approximation. For example, when used in conjunction with a numerical value, the terms can refer to a range of variation less than or equal to ±10% of that numerical value, such as less than or equal to ±5%, less than or equal to ±4%, less than or equal to ±3%, less than or equal to ±2%, less than or equal to ±1 %, less than or equal to ±0.5%, less than or equal to ±0.1 %, or less than or equal to ±0.05%. For example, two numerical values can be deemed to be "substantially" the same if a difference between the values is less than or equal to ±10% of an average of the values, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%.

Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range is explicitly specified.

While the present disclosure has been described and illustrated with primary to specific embodiments thereof, these descriptions and illustrations do not limit the present disclosure. It should be understood by those skilled in the art that various changes may be made and equivalents may be substituted without departing from the scope of the present invention as defined by the appended claims. The illustrations are not necessarily drawn to scale. There may be distinctions between the artistic renditions in the present disclosure and the actual apparatus due to manufacturing processes, tolerances and/or other reasons. There may be other embodiments of the present disclosure which are not specifically illustrated. The specification (other than the claims) and drawings are to be regarded as illustrative rather than restrictive. Modifications may be made to adapt a particular situation, material, composition of matter, technique, or process to the objective, without departing from the scope of the claims. All such modifications are intended to be within the scope of the claims appended hereto. While the techniques disclosed herein have been described with primary to particular operations performed in a particular order, it will be understood that these operations may be combined, sub-divided, or re-ordered to form an equivalent technique without departing from the teachings of the present disclosure. Accordingly, unless specifically indicated herein, the order and grouping of the operations are not limitations of the present disclosure.

## Claims

1. A method (600A) for characterizing and/or aligning product fill recipes, comprising:
generating (602A), by one or more flow sensors (104), first flow rate data (300) from a first product filling system over a plurality of first fill instances corresponding to a first product fill recipe;
generating (604A), by one or more processors (120), a plurality of first fill instance curves, wherein each first fill instance curve is a segment of the first flow rate data that corresponds to a different first fill instance of the plurality of first fill instances;
generating (606A), by the one or more processors, a first flow profile (408) based on the plurality of first fill instance curves;
the method **characterized by**:
generating (602B), by the one or more flow sensors or one or more other flow sensors, second flow rate data from a second product filling system over a plurality of second fill instances corresponding to a second product fill recipe;
generating (604B), by the one or more processors, a plurality of second fill instance curves, wherein each second fill instance curve is a segment of the second flow rate data that corresponds to a different second fill instance of the plurality of second fill instances;
generating (606B), by the one or more processors, a second flow profile based on the plurality of second fill instance curves;
determining, based on the first flow profile and the second flow profile, adjustments to one or more parameters of the second product fill recipe;
causing (608A), by the one or more processors, a display (124) to present the first flow profile;
causing (608B), by the one or more processors, the display to present the second flow profile; and
changing the one or more parameters of the second product fill recipe in accordance with the adjustments.

2. The method of claim 1, wherein the determining of the adjustments comprises comparing the first flow profile and the second flow profile to determine the adjustments.

3. The method of claim 1 or 2, comprising:
causing the display to simultaneously present the first flow profile and the second flow profile on a shared set of axes.

4. The method of any one of the preceding claims, wherein determining the adjustments includes determining the adjustments based on a Fréchet distance between the first flow profile and the second flow profile.

5. The method of any one of the preceding claims, further comprising:
replacing and/or modifying equipment of the second product filling system corresponding to the second product fill recipe in response to changing the one or more parameters of the second product fill recipe.

6. The method of any one of the preceding claims, wherein the one or more parameters are associated with one or more of: start pump dosing, dosing start ramp, dosing stop ramp, end pump dosing, drip retraction, or main drive speed.

7. The method of any one of the preceding claims, further comprising:
smoothing, by the one or more processors and before generating the plurality of first fill instance curves, the first flow rate data; and
smoothing, by the one or more processors and before generating the plurality of second fill instance curves, the second flow rate data.

8. The method of any one of the preceding claims, wherein generating the first flow profile includes using dynamic time warping.

9. The method of claim 8, wherein generating the first flow profile includes using dynamic time warping and averaging.

10. The method of claim 9, wherein generating the first flow profile includes using dynamic time warping and k-means averaging.

11. The method of any one of the preceding claims, wherein generating at least one of the first fill instance curves includes segmenting the first flow rate data based on one or more of: prominence, minimum spacing, minimum height, minimum threshold, or minimum width.

12. The method of any one of the preceding claims, wherein the plurality of first fill instances includes at least three first fill instances, and wherein generating the plurality of first fill instance curves includes excluding segments of the first flow rate data that correspond to a beginning first fill instance and an ending first fill instance.

13. The method of any one of the preceding claims, further comprising:
collecting new flow rate data from the first product filling system and the second product filling system (502A, 502B);
generating two new flow profiles corresponding to the flow rate data from each of the first product filling system and the second product filling system (504); and
comparing the two new flow profiles to confirm transferability between the first product filling system and the second product filling system (506).

14. A system (100) comprising:
one or more flow sensors (104);
one or more processors (120); and
one or more non-transitory, computer-readable media (128) storing instructions that, when executed by the one or more processors, cause the one or more processors to perform the method of any one of claims 1-13.

## Patentansprüche

1. Verfahren (600A) zum Charakterisieren und/oder Abstimmen von Produktfüllrezepturen, umfassend:
Generieren (602A), durch einen oder mehrere Durchflusssensoren (104), von ersten Durchflussmengendaten (300) von einem ersten Produktfüllsystem über eine Vielzahl von ersten Füllinstanzen, die einer ersten Produktfüllrezeptur entsprechen;
Generieren (604A), durch einen oder mehrere Prozessoren (120), einer Vielzahl von ersten Füllinstanzkurven, wobei jede erste Füllinstanzkurve ein Segment der ersten Durchflussmengendaten ist, die einer unterschiedlichen ersten Füllinstanz aus der Vielzahl von ersten Füllinstanzen entsprechen;
Generieren (606A), durch den einen oder die mehreren Prozessoren, eines ersten Durchflussprofils (408) basierend auf der Vielzahl von ersten Füllinstanzkurven;
Generieren (602B), durch den einen oder die mehreren Durchflusssensoren oder einen oder mehrere andere Durchflusssensoren, von zweiten Durchflussmengendaten von einem zweiten Produktfüllsystem über eine Vielzahl von zweiten Füllinstanzen, die einer zweiten Produktfüllrezeptur entsprechen;
Generieren (604B), durch den einen oder die mehreren Prozessoren, einer Vielzahl von zweiten Füllinstanzkurven, wobei jede zweite Füllinstanzkurve ein Segment der zweiten Durchflussmengendaten ist, die einer unterschiedlichen zweiten Füllinstanz aus der Vielzahl von zweiten Füllinstanzen entsprechen;
Generieren (606B), durch den einen oder die mehreren Prozessoren, eines zweiten Durchflussprofils basierend auf der Vielzahl von zweiten Füllinstanzkurven;
Bestimmen, basierend auf dem ersten Durchflussprofil und dem zweiten Durchflussprofil, von Anpassungen an einem oder mehreren Parametern der zweiten Produktfüllrezeptur;
Verursachen (608A), durch den einen oder die mehreren Prozessoren, dass eine Anzeige (124) das erste Durchflussprofil darstellt;
Verursachen (608B), durch den einen oder die mehreren Prozessoren, dass die Anzeige das zweite Durchflussprofil darstellt; und
Verändern des einen oder der mehreren Parameter der zweiten Produktfüllrezeptur gemäß den Anpassungen.

2. Verfahren nach Anspruch 1, wobei das Bestimmen der Anpassungen das Vergleichen des ersten Durchflussprofils und des zweiten Durchflussprofils umfasst, um die Anpassungen zu bestimmen.

3. Verfahren nach Anspruch 1 oder 2, umfassend:
Verursachen, dass die Anzeige simultan das erste Durchflussprofil und das zweite Durchflussprofil auf einem gemeinsamen Satz von Achsen darstellt.

4. Verfahren nach einem der vorhergehenden Ansprüche, wobei das Bestimmen der Anpassungen das Bestimmen der Anpassungen basierend auf einer Fréchet-Distanz zwischen dem ersten Durchflussprofil und dem zweiten Durchflussprofil beinhaltet.

5. Verfahren nach einem der vorhergehenden Ansprüche, ferner umfassend:
Ersetzen und/oder Modifizieren von Ausstattung des zweiten Produktfüllsystems entsprechend der zweiten Produktfüllrezeptur als Reaktion auf die Veränderung des einen oder der mehreren Parameter der zweiten Produktfüllrezeptur.

6. Verfahren nach einem der vorhergehenden Ansprüche, wobei der eine oder die mehreren Parameter mit einem oder mehreren aus Folgendem im Zusammenhang stehen: Startpumpendosierung, Dosierungsstartrampe, Dosierungsstopprampe, Endpumpendosierung, Tropfenrückzug oder Hauptantriebsgeschwindigkeit.

7. Verfahren nach einem der vorhergehenden Ansprüche, ferner umfassend:
Glätten, durch den einen oder die mehreren Prozessoren und vor dem Generieren der Vielzahl von ersten Füllinstanzkurven, der ersten Durchflussgeschwindigkeitsdaten; und
Glätten, durch den einen oder die mehreren Prozessoren und vor dem Generieren der Vielzahl von zweiten Füllinstanzkurven, der zweiten Durchflussgeschwindigkeitsdaten.

8. Verfahren nach einem der vorhergehenden Ansprüche, wobei das Generieren des ersten Durchflussprofils das Verwenden von Dynamic Time Warping beinhaltet.

9. Verfahren nach Anspruch 8, wobei das Generieren des ersten Durchflussprofils das Verwenden von Dynamic Time Warping und Mittelwertbildung beinhaltet.

10. Verfahren nach Anspruch 9, wobei das Generieren des ersten Durchflussprofils das Verwenden von Dynamic Time Warping und k-Means-Mittelwertbildung beinhaltet.

11. Verfahren nach einem der vorhergehenden Ansprüche, wobei das Generieren von mindestens einer aus den ersten Füllinstanzkurven das Segmentieren der ersten Durchflussgeschwindigkeitsdaten basierend auf einem oder mehreren aus Folgendem beinhaltet: Prominenz, Mindestabstand, Mindesthöhe, Mindestschwelle oder Mindestbreite.

12. Verfahren nach einem der vorhergehenden Ansprüche, wobei die Vielzahl der ersten Füllinstanzen mindestens drei erste Füllinstanzen beinhaltet, und wobei das Generieren der Vielzahl von ersten Füllinstanzkurven das Ausschließen von Segmenten der ersten Durchflussgeschwindigkeitsdaten beinhaltet, die einem Beginn der ersten Füllinstanz und einem Ende der ersten Füllinstanz entsprechen.

13. Verfahren nach einem der vorhergehenden Ansprüche, ferner umfassend:
Sammeln von neuen Durchflussgeschwindigkeitsdaten von dem ersten Produktfüllsystem und dem zweiten Produktfüllsystem (502A, 502B);
Generieren von zwei neuen Durchflussprofilen entsprechend den Durchflussgeschwindigkeitsdaten von jedem aus dem ersten Produktfüllsystem und dem zweiten Produktfüllsystem (504); und
Vergleichen der zwei neuen Durchflussprofile, um die Übertragbarkeit zwischen dem ersten Produktfüllsystem und dem zweiten Produktfüllsystem (506) zu bestätigen.

14. System (100), umfassend:
einen oder mehrere Durchflusssensoren (104);
einen oder mehrere Prozessoren (120); und
ein oder mehrere nichttransitorische, computerlesbare Medien (128), die Anweisungen speichern, die, wenn sie durch den einen oder die mehreren Prozessoren ausgeführt werden, verursachen, dass der eine oder die mehreren Prozessoren das Verfahren nach einem der Ansprüche 1-13 durchführt.

## Revendications

1. Procédé (600A) de caractérisation et/ou d'alignement de recettes de remplissage de produit, comprenant :
la génération (602A), par un ou plusieurs capteurs de débit (104), de premières données de débit (300) issues d'un premier système de remplissage de produit sur une pluralité de premières instances de remplissage correspondant à une première recette de remplissage de produit,
la génération (604A), par un ou plusieurs processeurs (120), d'une pluralité de premières courbes d'instance de remplissage, chaque première courbe d'instance de remplissage étant un segment des premières données de débit qui correspond à une première instance de remplissage différente de la pluralité de premières instances de remplissage,
la génération (606A), par le ou les processeurs, d'un premier profil de débit (408) en fonction de la pluralité des premières courbes d'instance de remplissage,
la génération (602B), par le ou les capteurs de débit ou par un ou plusieurs autres capteurs de débit, de deuxièmes données de débit issues d'un deuxième système de remplissage de produit sur une pluralité de deuxièmes instances de remplissage correspondant à une deuxième recette de remplissage de produit,
la génération (604B), par le ou les processeurs, d'une pluralité de deuxièmes courbes d'instance de remplissage, chaque deuxième courbe d'instance de remplissage étant un segment des deuxièmes données de débit qui correspond à une deuxième instance de remplissage différente de la pluralité de deuxièmes instances de remplissage,
la génération (606B), par le ou les processeurs, d'un deuxième profil de débit en fonction de la pluralité des deuxièmes courbes d'instance de remplissage,
la détermination, en fonction du premier profil de débit et du deuxième profil de débit, d'ajustements à apporter à un ou plusieurs paramètres de la deuxième recette de remplissage de produit,
la présentation (608A), sous l'effet du ou des processeurs, du premier profil de débit par un dispositif d'affichage (124),
la présentation (608B), sous l'effet du ou des processeurs, du deuxième profil de débit par le dispositif d'affichage, et
la modification du ou des paramètres de la deuxième recette de remplissage de produit conformément aux ajustements.

2. Procédé selon la revendication 1, dans lequel la détermination des ajustements comprend la comparaison du premier profil de débit et du deuxième profil de débit pour déterminer les ajustements.

3. Procédé selon la revendication 1 ou 2, comprenant :
la présentation simultanée, par le dispositif d'affichage, du premier profil de débit et du deuxième profil de débit sur un ensemble commun d'axes.

4. Procédé selon l'une quelconque des revendications précédentes, dans lequel la détermination des ajustements comprend la détermination des ajustements en fonction d'une distance de Fréchet entre le premier profil de débit et le deuxième profil de débit.

5. Procédé selon l'une quelconque des revendications précédentes, comprenant en outre :
le remplacement et/ou la modification d'un équipement du deuxième système de remplissage de produit correspondant à la deuxième recette de remplissage de produit en réaction à la modification du ou des paramètres de la deuxième recette de remplissage de produit.

6. Procédé selon l'une quelconque des revendications précédentes, dans lequel le ou les paramètres sont associés au dosage de pompe de démarrage, à la rampe de démarrage de dosage, à la rampe de fin de dosage, au dosage de pompe de fin, à la prévention des égouttures et/ou à la vitesse d'entraînement principale.

7. Procédé selon l'une quelconque des revendications précédentes, comprenant en outre :
le lissage, par le ou les processeurs et avant la génération de la pluralité de premières courbes d'instance de remplissage, des premières données de débit, et
le lissage, par le ou les processeurs et avant la génération de la pluralité de deuxièmes courbes d'instance de remplissage, des deuxièmes données de débit.

8. Procédé selon l'une quelconque des revendications précédentes, dans lequel la génération du premier profil de débit comprend l'utilisation d'un alignement temporel dynamique.

9. Procédé selon la revendication 8, dans lequel la génération du premier profil de débit comprend l'utilisation d'un alignement temporel dynamique et d'un moyennage.

10. Procédé selon la revendication 9, dans lequel la génération du premier profil de débit comprend l'utilisation d'un alignement temporal dynamique et d'un moyennage par l'algorithme des k-moyennes.

11. Procédé selon l'une quelconque des revendications précédentes, dans lequel la génération d'au moins l'une des premières courbes d'instance de remplissage comprend la segmentation des premières données de débit en fonction de la proéminence, de l'espacement minimal, de la hauteur minimale, du seuil minimal et/ou de la largeur minimale.

12. Procédé selon l'une quelconque des revendications précédentes, dans lequel la pluralité de premières instances de remplissage comprend au moins trois premières instances de remplissage, et dans lequel la génération de la pluralité de premières courbes d'instance de remplissage comprend l'exclusion de segments des premières données de débit qui correspondent à une première instance de remplissage initiale et à une première instance de remplissage finale.

13. Procédé selon l'une quelconque des revendications précédentes, comprenant en outre :
la collecte de nouvelles données de débit issues du premier système de remplissage de produit et du deuxième système de remplissage de produit (502A, 502B),
la génération de deux nouveaux profils de débit correspondant respectivement aux données de débit du premier système de remplissage de produit et du deuxième système de remplissage de produit (504), et
la comparaison des deux nouveaux profils de débit pour confirmer la transférabilité entre le premier système de remplissage de produit et le deuxième système de remplissage de produit (506).

14. Système (100) comprenant :
un ou plusieurs capteurs de débit (104),
un ou plusieurs processeurs (120), et
un ou plusieurs supports non transitoires lisibles par ordinateur (128) sur lesquels sont stockées des instructions qui, lorsqu'elles sont exécutées par le ou les processeurs, amènent le ou les processeurs à mettre en œuvre le procédé selon l'une quelconque des revendications 1 à 13.
